(19)

Europäisches
Patentamt
European
Patent Office
Office européen
des brevets

(11)  **EP 3 818 916 B1**

(12)  # EUROPEAN PATENT SPECIFICATION

(45) Date of publication and mention
of the grant of the patent:
**13.09.2023   Bulletin 2023/37**

(21) Application number: **19208260.0**

(22) Date of filing: **11.11.2019**

(51) International Patent Classification (IPC):
*A47J 36/00* (2006.01)          *G06Q 50/10* (2012.01)
*G16H 20/60* (2018.01)          *A47J 36/32* (2006.01)
*H05B 1/02* (2006.01)

(52) Cooperative Patent Classification (CPC):
**A47J 36/321; H05B 1/0261;** G16H 20/60

(54) **METHOD FOR PERFORMING A COOKING PROCESS ON THE BASIS OF A COOKING RECIPE INFORMATION**

VERFAHREN ZUR DURCHFÜHRUNG EINES KOCHPROZESSES AUF BASIS EINER KOCHREZEPTINFORMATION

PROCÉDÉ DE RÉALISATION D'UN PROCESSUS DE CUISSON SUR LA BASE D'INFORMATIONS DE RECETTES DE CUISINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**12.05.2021   Bulletin 2021/19**

(73) Proprietor: **ELECTROLUX APPLIANCES AKTIEBOLAG**
**105 45 Stockholm (SE)**

(72) Inventor: **LUCKHARDT, Christoph**
**91541 Rothenburg ob der Tauber (DE)**

(74) Representative: **Electrolux Group Patents**
**AB Electrolux**
**Group Patents**
**S:t Göransgatan 143**
**105 45 Stockholm (SE)**

(56) References cited:
**US-A1- 2015 185 712     US-A1- 2016 081 515**
**US-A1- 2016 220 064     US-A1- 2016 353 921**

**US-A1- 2017 011 649     US-A1- 2017 219 215**
**US-A1- 2019 150 652     US-A1- 2019 320 844**

- **Anonymous: "Free for", , 5 February 2019 (2019-02-05), XP055686145, Retrieved from the Internet:**
**URL:https://www.miele.de/media/ex/ce/press eartikel/2019/2019-004-the-new-wall-ovens-relaxed-to-culinary-delights.pdf [retrieved on 2020-04-16]**
- **Anonymous: "Whirlpool's smart oven identifies your food and lets you watch it cook in real time via an internal camera - The Verge", , 8 January 2019 (2019-01-08), XP055686141, Retrieved from the Internet:**
**URL:https://www.theverge.com/circuitbreake r/2019/1/8/18173569/smart-countertop-oven-whirlpool-wlabs-ces-2019 [retrieved on 2020-04-16]**
- **Anonymous: "WLabs(TM) of Whirlpool Corporation Cements Its Status as Innovator to Beat at CES 2019 | Whirlpool Corporation", , 3 January 2019 (2019-01-03), XP055686140, Retrieved from the Internet:**
**URL:https://whirlpoolcorp.com/wlabs-of-whi rlpool-corporation-cements-its-status-as-i nnovator-to-beat-at-ces-2019/ [retrieved on 2020-04-16]**

**Description**

The present invention relates to a method for performing a cooking process on the basis of a cooking recipe information. In particular, a food model is generated on the basis of the cooking recipe information and a database.

The users of cooking appliances obtain cooking recipes rather from the internet than from traditional cookery books. Internet sites like "allrecipies.com" or "chefkoch.de" provide a lot of cooking recipes submitted by users. Further, connected cooking appliances only offer the ability to use a predefined set of cooking recipes, since it is quite difficult to automatically import user submitted recipes and achieve good results. This is explained by the lack of information in the cooking recipes. For example, only a small part of the most popular cooking recipes of "chefkoch.de" states the cooking time in the intended field. Furthermore, information on the heating mode for cooking ovens is often missing in the cooking recipe. Moreover, predefined cooking recipes are just "set and go" without any adaption of the cooking process to the changes of the food during said cooking process.

The document US 2017/219215 A1 discloses a multi-sensored smart cooking apparatus.

The document US 2016/353921 A1 discloses a machine and system for obtaining precise cooking time of irregular shaped food objects.

The document US 2015/185712 A1 discloses a windows based gourmet maestro software commands a hardware controller to do the cooking process.

The document US 2019/320844 A1 discloses an apparatus generating a cooking process.

The document US 2017/011649 A1 discloses a recipe system.

The document Anonymous "Free for", XP055686145, discloses wall ovens.

The document Anonymous "smart oven identifies your food and lets you watch it cook in real time via an internal camera - The Verge", XP055686141, discloses a countertop oven having a built-in camera and object recognition to identify food.

The document XP055686140 discloses a smart countertop oven.

It is an object of the present invention to provide a method for performing a cooking process on the basis of an information receivable from a cooking recipe, which overcomes the problems mentioned above.

The object is achieved by the method according to claim 1.

According to the present invention a method for performing a cooking process on a food product ready to be cooked on the basis of a cooking recipe information is provided. The cooking recipe information is comprised in a cooking recipe. At least one food model is generated on the basis of the cooking recipe information and a database. The cooking recipe comprises at least one of the information about at least one ingredient, a preparation step or preparation process, the shape of unprocessed food and a composition, a property and/or condition of the food product.

The database comprises information about ingredients and/or unprocessed food which may be basic nutrient information of ingredients and/or unprocessed food. Particularly, the database comprises information about content of at least one of: water, fat, carbohydrates and proteins. Additionally or alternatively, the database comprises information about at least one thermal property of the unprocessed food or food product ready to be cooked.

If applicable, i. e. if not already directly available from and provided by the database, at least one thermal property of the food product ready to be cooked is estimated on the basis of the cooking recipe information and the basic nutrient information. Said thermal property of the food product may be its density, thermal conductivity and/or heat capacity. Based on the food model and/or on at least the provided or estimated at least one thermal property of the food product at least one cooking parameter of the cooking process is defined. The cooking parameter to be defined is particularly a temperature of the cooking equipment and/or cooking environment, the duration of a cooking program and/or of a program segment and/or of a program step, a heating mode, etc.

The composition of the food product may be extractable from the nutritional information the cooking recipe itself is providing. Alternatively, the composition may be calculable by adding up respective information about the individual ingredients.

The property of a food product may be an overall weight of a meal or a dish. Said overall weight may be calculable from a number of servings and individual nutritional information compared to the nutritional information for a reference weight, e.g. 100g.

The condition of the food product may be extractable from a food type category, which may be specific database information. For the example of cake dough, a derivable condition for a batter may be "low viscosity / high density", a derivable condition for a sponge cake mix may be "low viscosity / low density" and a derivable condition for a yeast dough may be "high viscosity /high density".

Specific ingredients may also enable derivation of influence on at least one of said composition, property and condition. In particular, if the ingredient "baking powder" is listed, a volume increase during the baking process may be expected.

The elements of the database may also be parameters of said ingredients and/or steps of the cooking process. Furthermore, the database may include information about cooking containers. Moreover, the database may include information about volume increase of certain ingredients by special ways of preparation, e.g. beating egg whites and/or about changes of the parameters and/or properties of the food during the cooking process.

The main target of the present invention is the completion of the cooking recipe, which target, according to the invention, is met under assistance of the at least one database. Empirically, a lot of cooking recipes are uncomplete or provided for another kind of cooking appliance. The present invention allows the use of arbitrary cooking recipes, which need not necessarily be complete, for an automatic, semiautomatic or manual cooking process.

The following terms herein before mentioned and herein after called shall be understood as follows. The term "unprocessed food" shall be understood as any kind of food or foodstuff not yet being prepared for cooking, particularly also covering meat or fish of raw nature. The term "food product ready to be cooked" shall be understood as any kind of food or foodstuff which is prepared by the chef or cook or other type of user of e. g. a cooking recipe and which is ready for a cooking process to be started. It includes prepared dough, casseroles, as well as unprocessed meat or fish which is e. g. salted and flavoured and waiting for the cooking process to be started. The single terms "food" or "food product" shall be understood as general terms for all kinds of food, foodstuff or food product, covering food product ready to be cooked as well as food product during the cooking process. The term "ingredient" shall be understood as all elements of a cooking recipe, i. e. all elements for preparing a food product, particularly such elements needed for the preparation of a dough or a casserole, but also unprocessed foodstuff like raw meat or fish if mentioned as a part or an element of a recipe.

The at least one cooking parameter of the cooking process may be defined in consideration of a cooking parameter proposal in the cooking recipe. Such proposal may be seen as a relevant indicator or only as a first reference point which needs a fine-tuning based on different kinds of parameters, particularly detected parameters or information about the food product.

A specific embodiment provides that the database includes information about a change of a cooking parameter and/or a property of an ingredient and/or of the food product during the cooking process and/or during the process of preparation of the food product. A cooking parameter may not be fixed during the whole cooking process but may be modified over time. Said modification over time may be dependent on specific changes of the food product during the cooking process.

According to embodiments, the food model is generated and/or adapted or updated during the cooking process by assistance of a user input and/or at least one detected parameter. The generation and/or adaptation or updating may be executed continuously or at discrete intervals. Additionally, the food model may be manipulated or manipulatable by an input of the user. Thus, the cooking process may be individually corrected by the user.

Preferably, the detected parameter comprises the shape of the food product ready to be cooked and/or the shape of the food product during the cooking process and/or the real temperature of the food product, in particular the real temperature of a specific zone of the food product, at a specific point in time during the cooking process. Said temperature is preferably measured by means of a food probe or any other type of thermometer.

The shape of the food product ready to be cooked and/or of the food product during the cooking process may be received with the aid of a scanning means, particularly a 3D scanner and/or a camera, and/or by means of information extracted from a description and/or creation instruction in the cooking recipe and/or by means of a user input. A 3D scanner and/or a camera making 3D images may be placed in an oven door handle and may take up information about e.g. unbaked bread inside of an oven compartment while the oven door is closed after food loading. A 3D scanner or a camera may also be located in a hood placed above a cooking hob and may take up information about a food product, e. g. a roast, inside a pan. Additionally or alternatively, the shape of the food product ready to be cooked may be extracted from a recipe description, particularly by means of information about a baking tin.

With said detected shape information, the food model may be constantly updated during cooking process with respect to such shape parameter. As an example, when a dough, e. g. of a bread, raises in the oven compartment, the thermal properties alter and the food model has to be re-calculated accordingly.

According to a specific embodiment, the food model is, particularly continuously or at discrete intervals, generated and/or adapted or updated during the cooking process in consideration of information about an ingredient which influences a desired effect or change in the food product prior to or during the cooking process. The database may comprise such information. Said desired effect or change in the food product is particularly denaturation of proteins (e. g. when heating tender meat or eggs), enzymatic activity (e. g. during treatment of tender and tough meat), hydrolysis of connective tissue (e. g. preferred when treating tough meat), formation of crumb structure and crust (e. g. when baking bread or cake), browning kinetics, drying, breakdown of cell walls (e. g. when heating fruits or vegetables), leavening (e. g. for receiving fluffy cake or bread), and/or killing of bacteria (e. g. meat, fish, etc.).

Particularly, the database comprises information about ingredients which affect further desired effects or changes. Said further effects may be received by means of sugar or egg yolk on surfaces during a baking process which influence the surface finish. Further, available sugar, in particular the amount thereof, for yeast (e. g. in a dough for a cake or bread) or an acid milieu for collagen (e. g. when cooking meat) may have a related effect during the cooking process.

The food model may be, particularly continuously or at discrete intervals, generated and/or adapted or updated during the cooking process in consideration of information about the amount of at least one gas, in particular $CO_2$ or air. Said gas or gases is incorporated in the food product due to a result or an effect of a preparation step or process and/or due to an influence of an ingredient and/or due to a chemical process. The amount or percentage or mass fraction of the at least one incorporated gas is preferably continuously adapted or updated during the cooking process.

According to embodiments, the at least one amount or percentage or mass fraction of the at least one gas is considered in at least one formula calculating the at least one estimated thermal property of the food product. This may be of help for a provision of a simple thermal model substance for homogenous food. Said amounts or percentages or mass fractions of gases are dependent on heat time, temperature and substrate and, preferably, are continuously adapted or updated over time during the cooking process.

In particular, at least one keyword is extracted from the cooking recipe and the food model is generated on the basis of the cooking recipe and at least one element of the database associated with said keyword, the database preferably including at least one concordance list, in which the keyword is associated with at least one element of the database. Said keyword is preferably used to categorize the food type, to choose the structure of the food model and to define the desired thermal effects. In particular, the food type may be chosen as being "homogenous" if the identified keyword is "mix" or "knead". The food type may be selected by "food with flat layers" in case of an identified keyword "stack" or "layer", or it may be selected by "circular layered food" if the keyword is "filling" or "stuffing". A desired thermal effect may be defined by "leavening" and/or "formation of a crumb and crust", if the keywords "bread" and "yeast" are identified.

According to embodiments, at least one phrase is extracted from the cooking recipe, wherein said phrase is associated with at least one element of the database, and wherein preferably said phrase is tabled in the concordance list and associated therein with the at least one element of the database and/or is associated with at least one cooking parameter. Said phrase may be taken from process steps or activities described in the cooking recipe. As an example, on the basis of an identified phrase like "egg whites stiff" or "beat until fluffy" the amount of an incorporated gases, e.g. carbon dioxide or air, is added to the sum of the estimated thermal properties of the food in order to provide a simple thermal model substance for homogenous food.

An estimation or determination of a thermal property by way of calculating with a formula may be provided. As is known form scientific literature (see formulas from Rizvi, S. S. H. and Rao, M. A. 1995, Engineering properties of food: CRC Press, 1995, Vol. 2nd Edition) the thermal conductivity $\lambda$ of the food may be determined by

$$\lambda = 0.25\ Xc + 0.155\ Xp + 0.16\ Xf + 0.135\ Xa + 0.58\ Xw,$$

wherein Xc is the mass fraction of carbohydrates, Xp is the mass fraction of protein, Xf is the mass fraction of fat, Xa is the mass fraction of ash and Xw is the mass fraction of water, and wherein the unit of the thermal conductivity $\lambda$ is W/(m·K) .

In a similar way, the specific heat capacity cp of the food may be determined by

$$cp = 1.424\ Xc + 1.549\ Xp + 1.675\ Xf + 0.837\ Xa + 4.187\ Xw,$$

wherein Xc is the mass fraction of carbohydrates, Xp is the mass fraction of protein, Xf is the mass fraction of fat, Xa is the mass fraction of ash and Xw is the mass fraction of water, and wherein the unit of the specific heat capacity cp is kJ/(kg·K).

Further, the density $\rho$ of the food may be determined by

$$\rho = 1.07\ Xc + 1.4\ Xp + 0.925\ Xf + 2.16\ Xa + Xw,$$

wherein Xc is the mass fraction of carbohydrates, Xp is the mass fraction of protein, Xf is the mass fraction of fat, Xa is the mass fraction of ash and Xw is the mass fraction of water, and wherein the unit of the density $\rho$ is kg/dm$^3$.

The equations mentioned above, according to the known determinations, allow the forecasting of the thermal conductivity $\lambda$, the specific heat capacity cp and the density $\rho$ of the food without any measure.

These known determinations, however, have been identified by the inventor as forming only a first approximation. The formulas lack important further factors of influence.

Hence, according to a specific embodiment, at least one thermal property is calculated by a sum comprising summands of multipliers with different mass fractions, particularly carbohydrate mass fractions, protein mass fractions, fat mass fractions, mineral mass fractions, water mass fractions and air mass fractions.

At least one of the summands may comprise a multiplier of carbohydrate mass fraction, fat mass fraction, water mass fraction or air mass fraction and may further comprise a multiplier of the thermal property of the same molecule, particularly biomolecule. As an example, when calculating the specific heat capacity of a specific food product, one summand of the formula may comprise a first multiplier of carbohydrate mass fraction and a second multiplier of the specific heat capacity of carbohydrate. The same may also apply for at least one of fat, water and air molecules.

Particularly, at least one thermal property of a water molecule and/or of an air gas mixture is a parabolic function over the temperature and at least one thermal property of a fat molecule and/or carbohydrate molecule is a linear function over the temperature.

According to specific embodiment, the following formulas may be applicable for calculating thermal properties of a food product.

```
cp = cp_carb*carb + a₁*prot + cp_fat*fat + a₂*mineral +
cp_water*water + cp_air*air;
```

```
k = k_carb*carb + b₁*prot + k_fat*fat + b₂*mineral +
k_water*water + k_air*air;
```

```
dens = dens_carb*carb + c₁*prot + dens_fat*fat + c₂*mineral +
dens_water*water + dens_air*air;
```

wherein k is the thermal conductivity [unit: W/(m*K)], cp is the specific heat capacity [unit: kJ/(kg*K)], dens is the density [unit: $kg/m^3$];

wherein $a_1$, $a_2$, $b_1$, $b_2$, $c_1$ and $c_2$ are rational numbers;

wherein carb is the mass fraction of carbohydrates, prot is the mass fraction of proteins, fat is the mass fraction of fat, mineral is the mass fraction of minerals, water is the mass fraction of water and air is the mass fraction of air;

and wherein

dens_air is the density of air and is calculated by:

```
dens_air = d₁*temp^2 + d₂*temp + d₃;
```

cp_air is the specific heat capacity of air and is calculated by:

```
cp_air = e₁*temp^2 + e₂*temp + e₃;
```

k_air is the thermal conductivity of air and is calculated by:

```
k_air = f₁*temp + f₂;
```

dens_carb is the density of carbohydrates and is calculated by:

```
dens_carb = g₁*temp+g₂;
```

cp_carb is the specific heat capacity of carbohydrates and is calculated by:

$$cp\_carb = (h_1*temp+h_2)/h_3;$$

k_carb is the thermal conductivity of carbohydrates and is calculated by:

$$k\_carb = i_1*temp + i_2;$$

dens_fat is the density of fat and is calculated by:

$$dens\_fat = j_1*temp + j_2;$$

cp_fat is the specific heat capacity of fat and is calculated by:

$$cp\_fat = (k_1*temp + k_2)/k_3;$$

k_fat is the thermal conductivity of fat and is calculated by:

$$k\_fat = l_1*temp + l_2;$$

dens_water is the density of water and is calculated by:

$$dens\_water = m_1*temp\string^2 + m_2*temp + m_3;$$

cp_water is the specific heat capacity of water and is calculated by:

$$cp\_water = (n_1*temp\string^2 + n_2*temp + n_3)/n_4;$$

k_water is the thermal conductivity of water and is calculated by:

$$k\_water = o_1*temp\string^2 + o_2*temp + o_3;$$

wherein $d_1$, $d_2$, $d_3$, $e_1$, $e_2$, $e_3$, $f_1$, $f_2$, $g_1$, $g_2$, $h_1$, $h_2$, $h_3$, $i_1$, $i_2$, $j_1$, $j_2$, $k_1$, $k_2$, $k_3$, $l_1$, $l_2$, $m_1$, $m_2$, $m_3$, $n_1$, $n_2$, $n_3$, $n_4$, $o_1$, $o_2$ and $o_3$ are rational numbers.

Finally, the thermal diffusivity a [unit: $m^2/s$] of a food product may be calculated by:

$$a=k/(dens*cp).$$

Preferably, the food model is adapted or adaptable to a specific cooking appliance by assistance of the database. Individual properties of the cooking appliance may be considered. For example, the heat transfer coefficients of the available cooking modes are considered. Particularly, the different oven types or models differ from each other in their features and functionalities. For a specific food product, according to a related recipe, there may be functionalities or feature required for a best cooking performance. If, however, such functionalities or features are not available in the user's oven, alternative features or functionalities may be used which might end up only with e. g. a second-best cooking performance.

In a preferred embodiment, the amount of an ingredient provided without weight specification is converted into a standardized weight. With such conversion, a calculation in weight units may be executed.

The described generation of at least one food model allows to "look inside of the food product" and the food model may be used to simulate different heat transfer scenarios, which may also be based on information from the oven, particularly the selected heating method, measured humidity or temperature inside of the oven compartment, etc.,

supporting the target to get the best result for the desired effects like those ones as described above.

Novel and inventive features of the present invention are set forth in the appended claims.

The present invention will be described in further detail with reference to the drawing, in which

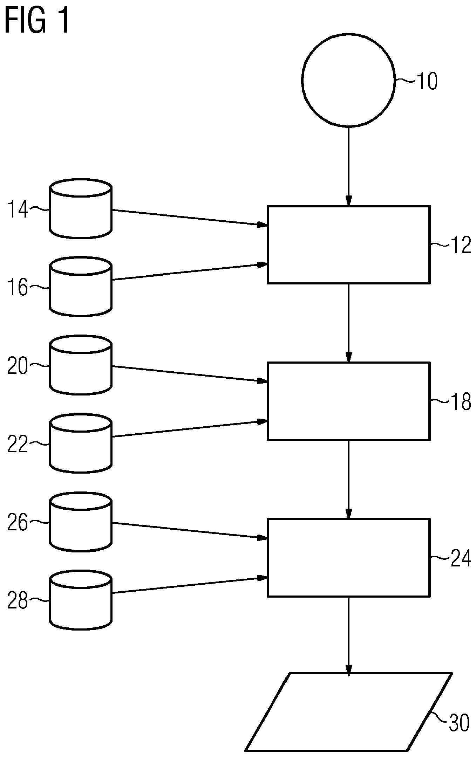

FIG 1    illustrates a schematic flow chart diagram of a method for performing a cooking process on the basis of a cooking recipe according to the present invention.

FIG 1 illustrates a schematic flow chart diagram of a method for performing a cooking process on the basis of a cooking recipe 10 according to the present invention. The cooking recipe 10 is downloaded from the internet and manipulated by a recipe deconstructor 12, a food model generator 18 and a cooking simulator 24.

The recipe deconstructor 12 is provided for scanning the cooking recipe for keywords. The recipe deconstructor 12 is connected to an ingredient database 14 and a cooking container database 16. The recipe deconstructor 12 provides further information about the food, e.g. the nutritional values, incorporated air and the shape by combing the extracted information from the recipe with the information of the connected databases 14, 16.

The food model generator 18 is connected to one or more conversion databases 20 and 22. The conversion databases 20 and 22 provide relationships between properties of the ingredients. The food model generator 18 considers information from the conversion databases 20 and 22.

The cooking simulator 24 is connected to an oven database 26 and a change database 28. The oven database 26 provides specific information about the used cooking oven and/or cooking hob. The change database 28 provides information about changes of the food during the cooking process. At last, a food model 30 is provided.

The food model 30 of the food described in the cooking recipe 10 is generated by evaluating the ingredients and processes. Since in most cooking recipes ingredients, time and temperature are used to achieve desired changes in or on the food, the food model 30 can be used to estimate best temperature, cooking time and heat transfer method in order to achieve the desired effects.

For example, some desired changes are a denaturation of proteins, e.g. in tender meat or egg, an enzymatic activity, e.g. in tender and tough meat, a hydrolysis of connective tissue, e.g. in tough meat, and a formation of crumb structure and crust, e.g. in bread or cake, browning kinetics, drying, breakdown of cell walls, e.g. in fruits or vegetables, leavening, e.g. in bread or cake, and killing of bacteria, e.g. in meat or fish.

The food model 30 according to the present invention is build up by comparing the ingredients in the cooking recipe 10 with a food database 14 containing the basic nutrient information, i.e. content of water, fat, carbohydrates and protein, in order to estimate the thermal and mechanical properties of the food, in particular density, heat conductivity and heat capacity.

Furthermore, the amount of the ingredients is converted to a standardized weight. For example, one egg has the standardized weight of 60 g and 100 ml oil has a standardized weight of 93 g.

On the basis of the ingredients like baking soda or yeast, the amount of incorporated gases, e.g. carbon dioxide or air, is added to the sum of the estimated thermal properties of the food. In a similar way, in processes described by phrases like "egg whites stiff" or "beat until fluffy", the amount of incorporated gases, e.g. carbon dioxide or air, is added to the sum of the estimated thermal properties of the food in order to provide a simple thermal model substance for homogenous food.

Since the amounts of ingredients depend on the time, heat and substrate, the fraction of gases has to be continuously adapted for the model. For example, yeast produces more carbon dioxide at an optimum temperature and nutrient supply, e.g. freely available sugars for yeast digestion.

Keywords in the cooking recipe 10 may be used to categorise the food type in order to choose the structure of the food model 30. Examples of said keywords in relation to structure are "mix" or "knead" may be related to homogenous structure, "stack" or "layer" may be related to food with flat layers, "filling" or "stuffing" may be related to circular layered food. Further, the keywords in the cooking recipe 10 may be used to define desired thermal effects. For example, the keywords "bread" and "yeast" result in leavening of dough and, further, in a formation of crumb and crust.

The shape of the food model 30 can also be extracted from the description of the cooking recipe 10, e.g. type of baking tin. This information may also be described by the user or 3D scanned from the ready to be cooked food. For example, unbaked bread is put into the cooking oven and 3D scanned by a camera in a door handle, while the oven door is closed. Further, a 3D scanner in a cooker hood may scan the shape of roast in a pan.

Then, the food model 30 can be used to simulate different heat transfer scenarios based on information from the cooking oven. For example, the heating method of the cooking oven and the humidity and temperature inside the oven cavity are available. The simulation of the different heat transfer scenarios allows that the best result for the desired effects is obtained. Furthermore, ingredients that considerably affect the desired changes, e.g. sugar, egg, yolk on surface, available sugar for yeast, acid milieu for hydrolysis of collagen, are considered within the food model 30.

The food model 30 for the method according to the present invention is explained by way of two examples relating to a bread recipe and a roast beef recipe.

First example relating to the bread recipe:

The user with a high-end steam oven having a 3D scanning camera system imports a bread recipe from an internet cooking portal, wherein no leavening time is stated. Further, the cooking time and temperature are stated for a gas cooking oven. On the basis of the list of ingredients a homogenous food model is generated. Since the list of ingredients contains yeast and the keyword "bread" is found in the recipe, the proposed cooking method is a three-phase baking program.

In the first phase the bread is proofed at low temperatures with an initial steam boost in order to make the surface flexible and solve some starch to generate a starch water solution which develops a glossy crust during baking.

The second phase is the baking phase at high humidity and high temperature in order to get a closed crust and a fluffy crumb.

The third phase is performed at medium humidity and medium to high temperature in order to finish the bread based on the desired crust input of the user.

On the basis of the ingredients, e.g. amount of yeast or available sugars, the optimal cooking time, the temperature and the humidity of the first phase are set. The food model is constantly adapted due to the generation of gases, particularly air. The air is considered for the density, conductivity and heat capacity. Moreover, the air or other kind of gas is considered for adapting the shape of the food, e.g. spherical expansion or growing upward in tin. During the proofing of the bread the 3D scanning camera system monitors the growth of the dough.

The second phase is started when the desired volume of the bread is reached. The food model is used to calculate the change of the temperature distribution inside the bread based on gathered sensor information, e.g. oven temperature, humidity and/or shrinking of bread based on camera image, and the food model. An estimation of the time needed to achieve a full crumb formation is done.

The third phase is started before the crumb formation is finished. Depending on the desired crust input the cooking time and temperature of the third phase is set.

Second example relating to the roast beef recipe:

The user puts the roast beef into the oven cavity of the cooking oven and provides via application software or the user interface that the inserted food is a roast beef.

From the database the thermal properties of the roast beef are extracted. The user is asked to stick the food probe into the roast beef. The user is asked for the desired cooking degree of said roast beef and when the food should be finished.

The cooking oven starts heating the food. The size of the food is extracted on the basis of the thermal flow from the outside to the inside of said food, wherein the parameters on the outside are known from the cooking oven, while the parameters in the inside are known from the database and the food probe. The food model is built on the basis of the available data.

This food model is then used to obtain the best result in the time given by the user, by heating up the food to an optimal temperature depending on desired cooking degree. For the enzymatic activity, e.g. 40°C to 60°C for sarcoplasmic enzymes, the temperature is hold as long as possible. For example, the food model 30 for meat depends on the degree of "toughness". In turn, said toughness depends substantially on type and age of said meat. For already tender meat according to "Modernist Cuisine" it is sufficient to reach the final temperature. For example, a medium tough cut like a flank can be kept at a temperature of 50°C for three hours to get a firm texture, a temperature of 55°C for twelve hours to get a tender texture and a temperature of 62°C for thirty-six hours to get a flaky texture.

## Claims

1. A method for performing a cooking process on a food product ready to be cooked on the basis of a cooking recipe information provided in a cooking recipe (10), wherein the method comprises:

   - downloading the cooking recipe (10) from the internet; and
   - manipulating the cooking recipe (10) by means of a recipe deconstructor (12), a food model generator (18) and a cooking simulator (24);
   wherein the recipe deconstructor (12) scans the cooking recipe (10) for keywords and is connected to an ingredient database (14) and a cooking container database (16);
   wherein the recipe deconstructor (12) provides further information about the food by combing extracted information from the cooking recipe (10) with the information of the connected ingredient database (14) and the cooking container database (16) ;

wherein the food model generator (18) is connected to one or more conversion databases (20, 22) providing relationships between properties of ingredients and the food model generator (18) considers information from the conversion databases (20, 22);

wherein the cooking simulator (24) is connected to an oven database (26) providing specific information about a used cooking oven and/or cooking hob and a change database (28) providing information about changes of the food during the cooking process;

wherein:

- at least one food model (30) is generated on the basis of the cooking recipe information and a database (14, 16, 20, 22, 26, 28) comprising the ingredient database (14), the cooking container database (16), the one or more conversion databases (20, 22), the oven database (26) and the change database (28);

the method also comprises a step of simulating, during which the food model (30) is used to simulate different heat transfer scenarios based on information from a cooking oven;

- the cooking recipe (10) comprises at least one of the information about:

  • an ingredient,
  • a preparation step or preparation process,
  • the shape of unprocessed food, and
  • composition, properties and condition of the food product

- the database (14, 16, 20, 22, 26, 28) comprises information about ingredients and/or unprocessed food, in particular basic nutrient information of ingredients and/or unprocessed food, particularly information about content of at least one of: water, fat, carbohydrates and proteins, and/or information about at least one thermal property of the unprocessed food or food product ready to be cooked,
- at least one thermal property of the food product ready to be cooked, in particular its density, thermal conductivity and/or heat capacity, is estimated on the basis of the cooking recipe information and the basic nutrient information, if not already directly available from and provided by the database (14, 16, 20, 22, 26, 28), and
- at least one cooking parameter of the cooking process, in particular temperature of the cooking equipment and/or environment, duration of a cooking program and/or program segment and/or program step, heating mode, is defined based on at least the provided or estimated at least one thermal property of the food product.

2. The method according to claim 1,
   **characterised in that**
   the database (14, 16, 20, 22, 26, 28) includes information about a change of a cooking parameter and/or a property of an ingredient and/or of the food product during the cooking process and/or during the process of preparation of the food product ready to be cooked.

3. The method according to claim 1 or 2,
   **characterised in that**
   the food model (30) is, particularly continuously or at discrete intervals, generated and/or adapted or updated during the cooking process by assistance of a user input and/or at least one detected parameter.

4. The method according to claim 3,
   **characterised in that**
   the detected parameter comprises the shape of the food product ready to be cooked and/or the shape of the food product during the cooking process and/or the real temperature of the food product at a specific point in time during the cooking process, in particular the real temperature of a specific zone of the food product, preferably measured by means of a food probe or any other type of thermometer.

5. The method according to claim 4,
   **characterized in that**
   the shape of the food product ready to be cooked and/or of the food product during the cooking process is received by means of a scanning means, particularly a 3D scanner and/or a camera, and/or by means of information extracted from a description and/or creation instruction in the cooking recipe (10) and/or by means of a user input.

**6.** The method according to anyone of the preceding claims,
**characterized in that**
the food model (30) is, particularly continuously or at discrete intervals, generated and/or adapted or updated during the cooking process in consideration of information about an ingredient which influences a desired effect or change in the food product prior to or during the cooking process, particularly denaturation of proteins, enzymatic activity, hydrolysis of connective tissue, formation of crumb structure and crust, browning kinetics, drying, breakdown of cell walls, leavening, and/or killing of bacteria.

**7.** The method according to anyone of the preceding claims,
**characterized in that**
the food model (30) is, particularly continuously or at discrete intervals, generated and/or adapted or updated during the cooking process in consideration of information about the amount or ratio of at least one gas, in particular $CO_2$ or air, which is incorporated in the food product due to a result or an effect of a preparation step or process and/or due to an influence of an ingredient and/or due to a chemical process, the amount or percentage or mass fraction of the at least one gas preferably being continuously adapted or updated during the cooking process.

**8.** The method according to claim 7,
**characterized in that**
the at least one amount or percentage or mass fraction of the at least one gas is considered in at least one formula calculating the at least one estimated thermal property of the food product.

**9.** The method according to any one of the preceding claims, **characterised in that**
at least one keyword is extracted from the cooking recipe (10) and the food model (30) is generated on the basis of the cooking recipe (10) and at least one element of the database (14, 16, 20, 22, 26, 28) associated with the keyword, the database (14, 16, 20, 22, 26, 28) preferably including at least one concordance list, in which the keyword is associated with at least one element of the database (14, 16, 20, 22, 26, 28) .

**10.** The method according to anyone of the preceding claims,
**characterized in that**
at least one phrase is extracted from the cooking recipe (10), wherein said phrase is associated with at least one element of the database (14, 16, 20, 22, 26, 28), and wherein preferably said phrase is tabled in the concordance list and associated therein with the at least one element of the database (14, 16, 20, 22, 26, 28) and/or is associated with at least one cooking parameter.

**11.** The method according to any one of the preceding claims,
**characterised in that**
the at least one thermal property is calculated by a sum comprising summands of multipliers with different mass fractions, particularly carbohydrate mass fractions, protein mass fractions, fat mass fractions, mineral mass fractions, water mass fractions and air mass fractions.

**12.** The method according to claim 11,
**characterised in that**
at least one of the summands comprises a multiplier of carbohydrate mass fraction, fat mass fraction, water mass fraction or air mass fraction and further comprises a multiplier of the thermal property of the same molecule, particularly biomolecule.

**13.** The method according to claim 12,
**characterised in that**
at least one thermal property of a water molecule and/or of an air gas mixture is a parabolic function over the temperature and at least one thermal property of a fat molecule and/or carbohydrate molecule is a linear function over the temperature.

**14.** The method according to any one of the preceding claims,
**characterised in that**
the food model (30) is adapted or adaptable to a specific cooking appliance by assistance of the database (14, 16, 20, 22, 26, 28).

**15.** The method according to any one of the preceding claims,

**characterised in that**
the amount of an ingredient provided without weight specification is converted into a standardized weight.

**Patentansprüche**

1. Verfahren zur Durchführung eines Kochprozesses bezüglich eines kochfertigen Lebensmittelprodukts auf Basis einer Kochrezeptinformation, die in einem Kochrezept (10) bereitgestellt ist, wobei das Verfahren Folgendes umfasst:

   - Herunterladen des Kochrezepts (10) aus dem Internet; und
   - Bearbeiten des Kochrezepts (10) mittels eines Rezeptzergliederers (12), eines Lebensmittelmodellgenerators (18) und eines Kochsimulators (24);
   wobei der Rezeptzergliederer (12) das Kochrezept (10) nach Schlüsselwörtern durchsucht und mit einer Zutatendatenbank (14) und einer Kochbehälterdatenbank (16) verbunden ist;
   wobei der Rezeptzergliederer (12) weitere Informationen über das Lebensmittel bereitstellt, indem er aus dem Kochrezept (10) extrahierte Informationen mit den Informationen der mit ihm verbundenen Zutatendatenbank (14) und der Kochbehälterdatenbank (16) kombiniert;
   wobei der Lebensmittelmodellgenerator (18) mit einer oder mehreren Umrechnungsdatenbanken (20, 22) verbunden ist, die Beziehungen zwischen Eigenschaften von Zutaten bereitstellen, und der Lebensmittelmodellgenerator (18) Informationen aus den Umrechnungsdatenbanken (20, 22) berücksichtigt;
   wobei der Kochsimulator (24) mit einer Herddatenbank (26), die spezifische Informationen über eine(n) verwendeten Kochherd und/oder Kochfläche bereitstellt, und einer Änderungsdatenbank (28) die Informationen über Veränderungen des Lebensmittels während des Kochprozesses bereitstellt, verbunden ist;
   wobei:

      - wenigstens ein Lebensmittelmodell (30) auf Basis der Kochrezeptinformation und einer Datenbank (14, 16, 20, 22, 26, 28), welche die Zutatendatenbank (14), die Kochbehälterdatenbank (16), die eine oder mehreren Umrechnungsdatenbanken (20, 22), die Herddatenbank (26) und die Änderungsdatenbank (28) umfasst, erzeugt wird;

   das Verfahren auch einen Schritt des Simulierens umfasst, während dessen das Lebensmittelmodell (30) dazu verwendet wird, basierend auf Informationen von einem Kochherd verschiedene Wärmeübertragungsszenarios zu simulieren; - das Kochrezept (10) wenigstens eine der Informationen über Folgendes umfasst:

      • eine Zutat,
      • einen Zubereitungsschritt oder einen Zubereitungsprozess,
      • die Form des unverarbeiteten Lebensmittels und
      • die Zusammensetzung, die Eigenschaften und den Zustand des Lebensmittelprodukts,

   - die Datenbank (14, 16, 20, 22, 26, 28) Informationen über Zutaten und/oder unverarbeitete Lebensmittel, insbesondere grundlegende Nährwertangaben von Zutaten und/oder unverarbeiteten Lebensmitteln, insbesondere Informationen über den Gehalt an mindestens einem der Folgenden: Wasser, Fett, Kohlenhydrate und Eiweiße, und/oder Informationen über wenigstens eine thermische Eigenschaft des unverarbeiteten Lebensmittels oder des kochfertigen Lebensmittelprodukts umfasst,
   - wenigstens eine thermische Eigenschaft des kochfertigen Lebensmittelprodukts, insbesondere seine Dichte, Wärmeleitfähigkeit und/oder Wärmekapazität, auf Basis der Kochrezeptinformation und der grundlegenden Nährwertangaben geschätzt wird, wenn sie nicht bereits direkt aus der Datenbank (14, 16, 20, 22, 26, 28) verfügbar ist und durch diese bereitgestellt wird, und
   - wenigstens ein Kochparameter des Kochprozesses, insbesondere die Temperatur der Kocheinrichtung und/oder -umgebung, die Dauer eines Kochprogramms und/oder -programmabschnitts und/oder -programmschritts, der Heizmodus, basierend auf wenigstens der bereitgestellten oder geschätzten wenigstens einen thermischen Eigenschaft des Lebensmittelprodukts definiert wird.

2. Verfahren nach Anspruch 1,
   **dadurch gekennzeichnet, dass**
   die Datenbank (14, 16, 20, 22, 26, 28) Informationen über eine Änderung eines Kochparameters und/oder einer Eigenschaft einer Zutat und/oder des Lebensmittelprodukts während des Kochprozesses und/oder während des Prozesses der Zubereitung des kochfertigen Lebensmittelprodukts enthält.

3. Verfahren nach Anspruch 1 oder 2,
**dadurch gekennzeichnet, dass**
das Lebensmittelmodell (30) während des Kochprozesses, insbesondere kontinuierlich oder in diskreten Abständen, mithilfe einer Benutzereingabe und/oder wenigstens eines erfassten Parameters erzeugt und/oder angepasst oder aktualisiert wird.

4. Verfahren nach Anspruch 3,
**dadurch gekennzeichnet, dass**
der erfasste Parameter die Form des kochfertigen Lebensmittelprodukts und/oder die Form des Lebensmittelprodukts während des Kochprozesses und/oder die tatsächliche Temperatur des Lebensmittelprodukts zu einem spezifischen Zeitpunkt während des Kochprozesses, insbesondere die tatsächliche Temperatur eines spezifischen Bereichs des Lebensmittelprodukts, die vorzugsweise mittels eines Speisenthermometers oder einer beliebigen anderen Art von Thermometer gemessen wird, umfasst.

5. Verfahren nach Anspruch 4,
**dadurch gekennzeichnet, dass**
die Form des kochfertigen Lebensmittelprodukts und/oder des Lebensmittelprodukts während des Kochprozesses über ein Abtastmittel, insbesondere einen 3D-Scanner und/oder eine Kamera, und/oder über aus einer Beschreibung und/oder Herstellungsanweisung in dem Kochrezept (10) extrahierte Informationen und/oder über eine Benutzereingabe empfangen wird.

6. Verfahren nach einem der vorangehenden Ansprüche, **dadurch gekennzeichnet, dass**
das Lebensmittelmodell (30) während des Kochprozesses, insbesondere kontinuierlich oder in diskreten Abständen, erzeugt und/oder angepasst oder aktualisiert wird, und zwar unter Berücksichtigung von Informationen über eine Zutat, die eine(n) gewünschte(n) Effekt oder Veränderung in dem Lebensmittelprodukt vor oder während des Kochprozesses, insbesondere die Denaturierung von Eiweißen, die enzymatische Aktivität, die Hydrolyse von Bindegewebe, die Ausbildung einer Krumenstruktur und einer Kruste, die Bräunungskinetik, die Trocknung, das Aufbrechen von Zellwänden, die Lockerung und/oder die Abtötung von Bakterien, beeinflusst.

7. Verfahren nach einem der vorangehenden Ansprüche, **dadurch gekennzeichnet, dass**
das Lebensmittelmodell (30) während des Kochprozesses, insbesondere kontinuierlich oder in diskreten Abständen, erzeugt und/oder angepasst oder aktualisiert wird, und zwar unter Berücksichtigung von Informationen über die Menge oder den Anteil wenigstens eines Gases, insbesondere $CO_2$ oder Luft, die/der aufgrund eines Ergebnisses oder eines Effekts eines Zubereitungsschritts oder -prozesses und/oder aufgrund eines Einflusses einer Zutat und/oder aufgrund eines chemischen Prozesses in das Lebensmittelprodukt aufgenommen wird, wobei die Menge oder der Prozentsatz oder der Massenanteil des wenigstens einen Gases vorzugsweise während des Kochprozesses kontinuierlich angepasst oder aktualisiert wird.

8. Verfahren nach Anspruch 7,
**dadurch gekennzeichnet, dass**
die/der wenigstens eine Menge oder Prozentsatz oder Massenanteil des wenigstens einen Gases in wenigstens einer Formel zum Berechnen der wenigstens einen geschätzten thermischen Eigenschaft des Lebensmittelprodukts berücksichtigt wird.

9. Verfahren nach einem der vorangehenden Ansprüche, **dadurch gekennzeichnet, dass**
wenigstens ein Schlüsselwort aus dem Kochrezept (10) extrahiert wird und das Lebensmittelmodell (30) auf Basis des Kochrezepts (10) und wenigstens eines Elements der Datenbank (14, 16, 20, 22, 26, 28), das dem Schlüsselwort zugeordnet ist, erzeugt wird, wobei die Datenbank (14, 16, 20, 22, 26, 28) vorzugsweise wenigstens eine Konkordanzliste enthält, in der das Schlüsselwort wenigstens einem Element der Datenbank (14, 16, 20, 22, 26, 28) zugeordnet ist.

10. Verfahren nach einem der vorangehenden Ansprüche, **dadurch gekennzeichnet, dass**
wenigstens eine Formulierung aus dem Kochrezept (10) extrahiert wird, wobei die Formulierung wenigstens einem Element der Datenbank (14, 16, 20, 22, 26, 28) zugeordnet ist und wobei vorzugsweise die Formulierung in der Konkordanzliste aufgeführt und darin dem wenigstens einen Element der Datenbank (14, 16, 20, 22, 26, 28) zugeordnet ist und/oder wenigstens einem Kochparameter zugeordnet ist.

11. Verfahren nach einem der vorangehenden Ansprüche, **dadurch gekennzeichnet, dass**

die wenigstens eine thermische Eigenschaft durch eine Summe berechnet wird, die Summanden aus Multiplikatoren und verschiedenen Massenanteilen, insbesondere Kohlenhydrat-Massenanteilen, Eiweiß-Massenanteilen, Fett-Massenanteilen, Mineralstoff-Massenanteilen, Wasser-Massenanteilen und Luft-Massenanteilen, umfasst.

**12.** Verfahren nach Anspruch 11,
**dadurch gekennzeichnet, dass**
wenigstens einer der Summanden einen Multiplikator des Kohlenhydrat-Massenanteils, des Fett-Massenanteils, des Wasser-Massenanteils oder des Luft-Massenanteils umfasst und ferner einen Multiplikator der thermischen Eigenschaft desselben Moleküls, insbesondere Biomoleküls, umfasst.

**13.** Verfahren nach Anspruch 12,
**dadurch gekennzeichnet, dass**
wenigstens eine thermische Eigenschaft eines Wassermoleküls und/oder eines Luft/Gas-Gemischs eine Parabelfunktion über der Temperatur ist und wenigstens eine thermische Eigenschaft eines Fettmoleküls und/oder eines Kohlenhydratmoleküls eine lineare Funktion über der Temperatur ist.

**14.** Verfahren nach einem der vorangehenden Ansprüche, **dadurch gekennzeichnet, dass**
das Lebensmittelmodell (30) mithilfe der Datenbank (14, 16, 20, 22, 26, 28) an ein spezifisches Kochgerät angepasst wird oder anpassbar ist.

**15.** Verfahren nach einem der vorangehenden Ansprüche, **dadurch gekennzeichnet, dass**
die Menge einer Zutat, die ohne Gewichtsangabe angegeben ist, in ein standardisiertes Gewicht umgerechnet wird.

## Revendications

**1.** Procédé destiné à réaliser un processus de cuisson sur un produit alimentaire prêt à être cuit sur la base d'informations de recette de cuisine fournie dans une recette de cuisine (10), le procédé comprenant :

- le téléchargement de la recette de cuisine (10) à partir d'Internet ; et
- la manipulation de la recette de cuisine (10) au moyen d'un déconstructeur de recettes (12), d'un générateur de modèles alimentaires (18) et d'un simulateur de cuisson (24) ;
le déconstructeur de recettes (12) recherchant des mots-clés dans la recette de cuisine (10) et étant connecté à une base de données d'ingrédients (14) et à une base de données de récipients de cuisson (16) ; le déconstructeur de recettes (12) fournissant des informations supplémentaires sur l'aliment en combinant des informations extraites de la recette de cuisine (10) avec les informations de la base de données d'ingrédients (14) et de la base de données de récipients de cuisson (16) connectées ;
le générateur de modèles alimentaires (18) étant connecté à une ou plusieurs bases de données de conversion (20, 22) fournissant des relations entre des propriétés des ingrédients, et le générateur de modèles alimentaires (18) prenant en compte des informations provenant des bases de données de conversion (20, 22) ;
le simulateur de cuisson (24) étant connecté à une base de données de fours (26) fournissant des informations spécifiques sur un four et/ou une plaque de cuisson utilisés et à une base de données de changements (28) fournissant des informations sur les changements de l'aliment pendant le processus de cuisson ;

- au moins un modèle alimentaire (30) étant généré sur la base des informations de recette de cuisine et d'une base de données (14, 16, 20, 22, 26, 28) comprenant la base de données d'ingrédients (14), la base de données de récipients de cuisson (16), la ou les bases de données de conversion (20, 22), la base de données de fours (26) et la base de données de changements (28) ; le procédé comprenant également une étape de simulation, au cours de laquelle le modèle alimentaire (30) est utilisé pour simuler différents scénarios de transfert de chaleur sur la base d'informations provenant d'un four de cuisson ;
- la recette de cuisine (10) comprenant au moins l'une des informations suivantes :

  ◦ un ingrédient,
  ◦ une étape de préparation ou un processus de préparation,
  ◦ la forme de l'aliment non transformé, et
  ◦ la composition, les propriétés et l'état du produit alimentaire

- la base de données (14, 16, 20, 22, 26, 28) comprenant des informations sur les ingrédients et/ou les denrées alimentaires non transformées, en particulier des informations nutritionnelles de base sur les ingrédients et/ou les denrées alimentaires non transformées, en particulier des informations sur la teneur en au moins un des éléments suivants : eau, lipides, glucides et protéines, et/ou des informations sur au moins une propriété thermique de la denrée alimentaire non transformée ou du produit alimentaire prêt à être cuit,

- au moins une propriété thermique du produit alimentaire prêt à être cuit, en particulier sa densité, sa conductivité thermique et/ou sa capacité thermique, étant estimée sur la base des informations de recette de cuisine et des informations nutritionnelles de base, si elles ne sont pas déjà directement disponibles et fournies par la base de données (14, 16, 20, 22, 26, 28), et

- au moins un paramètre de cuisson du processus de cuisson, en particulier la température de l'équipement de cuisson et/ou de l'environnement, la durée d'un programme de cuisson et/ou d'un segment de programme et/ou d'une étape de programme, le mode de chauffage, étant défini sur la base d'au moins une propriété thermique fournie ou estimée du produit alimentaire.

2. Procédé selon la revendication 1,
**caractérisé en ce que**
la base de données (14, 16, 20, 22, 26, 28) comprend des informations sur le changement d'un paramètre de cuisson et/ou d'une propriété d'un ingrédient et/ou du produit alimentaire pendant le processus de cuisson et/ou pendant le processus de préparation du produit alimentaire prêt à être cuit.

3. Procédé selon la revendication 1 ou 2,
**caractérisé en ce que**
le modèle alimentaire (30) est, en particulier en continu ou à intervalles discrets, généré et/ou adapté ou mis à jour pendant le processus de cuisson à l'aide d'une entrée utilisateur et/ou d'au moins un paramètre détecté.

4. Procédé selon la revendication 3,
**caractérisé en ce que**
le paramètre détecté comprend la forme du produit alimentaire prêt à être cuit et/ou la forme du produit alimentaire pendant le processus de cuisson et/ou la température réelle du produit alimentaire à un moment spécifique du processus de cuisson, en particulier la température réelle d'une zone spécifique du produit alimentaire, de préférence mesurée au moyen d'une sonde alimentaire ou de tout autre type de thermomètre.

5. Procédé selon la revendication 4,
**caractérisé en ce que**
la forme du produit alimentaire prêt à être cuit et/ou du produit alimentaire pendant le processus de cuisson est reçue à l'aide d'un moyen de balayage, en particulier un scanner 3D et/ou une caméra, et/ou à l'aide d'informations extraites d'une description et/ou d'une instruction de création dans la recette de cuisine (10) et/ou à l'aide d'une entrée de l'utilisateur.

6. Procédé selon l'une quelconque des revendications précédentes,
**caractérisé en ce que**
le modèle alimentaire (30) est, en particulier en continu ou à intervalles discrets, généré et/ou adapté ou mis à jour pendant le processus de cuisson en tenant compte d'informations sur un ingrédient qui influence un effet ou un changement souhaité dans le produit alimentaire avant ou pendant le processus de cuisson, en particulier la dénaturation des protéines, l'activité enzymatique, l'hydrolyse du tissu conjonctif, la formation de la structure de la mie et de la croûte, la cinétique de brunissement, le séchage, la décomposition des parois cellulaires, le levage, et/ou la destruction des bactéries.

7. Procédé selon l'une quelconque des revendications précédentes,
**caractérisé en ce que**
le modèle alimentaire (30) est, en particulier en continu ou à intervalles discrets, généré et/ou adapté ou mis à jour pendant le processus de cuisson en tenant compte d'informations sur la quantité ou le rapport d'au moins un gaz, en particulier le $CO_2$ ou l'air, qui est incorporé dans le produit alimentaire en raison d'un résultat ou d'un effet d'une étape ou d'un processus de préparation et/ou en raison d'une influence d'un ingrédient et/ou en raison d'un processus chimique, la quantité ou le pourcentage ou la fraction massique de l'au moins un gaz étant de préférence adapté ou mis à jour en continu pendant le processus de cuisson.

8. Procédé selon la revendication 7,

**caractérisé en ce que**

l'au moins une quantité ou un pourcentage ou une fraction massique de l'au moins un gaz est pris en compte dans au moins une formule calculant l'au moins une propriété thermique estimée du produit alimentaire.

9. Procédé selon l'une quelconque des revendications précédentes,
**caractérisé en ce que**
au moins un mot-clé est extrait de la recette de cuisine (10) et le modèle alimentaire (30) est généré à partir de la recette de cuisine (10) et d'au moins un élément de la base de données (14, 16, 20, 22, 26, 28) associé au mot-clé, la base de données (14, 16, 20, 22, 26, 28) comprenant de préférence au moins une liste de concordance, le mot-clé étant associé à au moins un élément de la base de données (14, 16, 20, 22, 26, 28).

10. Procédé selon l'une quelconque des revendications précédentes,
**caractérisé en ce que**
au moins une phrase est extraite de la recette de cuisine (10), ladite phrase étant associée à au moins un élément de la base de données (14, 16, 20, 22, 26, 28) et, de préférence, ladite phrase est classée dans la liste de concordance et associée à au moins un élément de la base de données (14, 16, 20, 22, 26, 28) et/ou est associée à au moins un paramètre de cuisson.

11. Procédé selon l'une quelconque des revendications précédentes,
**caractérisé en ce que**
l'au moins une propriété thermique est calculée par une somme comprenant des sommations de multiples de différentes fractions de masse, en particulier des fractions de masse de glucides, des fractions de masse de protéines, des fractions de masse de lipides, des fractions de masse de minéraux, des fractions de masse d'eau et des fractions de masse d'air.

12. Procédé selon la revendication 11,
**caractérisé en ce que**
au moins une des sommations comprend un multiple de la fraction massique des glucides, de la fraction massique des lipides, de la fraction massique de l'eau ou de la fraction massique de l'air et comprend en outre un multiple de la propriété thermique de la même molécule, en particulier d'une biomolécule.

13. Procédé selon la revendication 12,
**caractérisé en ce que**
au moins une propriété thermique d'une molécule d'eau et/ou d'un mélange gazeux d'air est une fonction parabolique de la température et au moins une propriété thermique d'une molécule de lipides et/ou d'une molécule de glucides est une fonction linéaire de la température.

14. Procédé selon l'une quelconque des revendications précédentes,
**caractérisé en ce que**
le modèle alimentaire (30) est adapté ou adaptable à un appareil de cuisson spécifique à l'aide de la base de données (14, 16, 20, 22, 26, 28).

15. Procédé selon l'une quelconque des revendications précédentes,
**caractérisé en ce que**
la quantité d'un ingrédient fourni sans spécification de poids est convertie en un poids standardisé.

FIG 1

## REFERENCES CITED IN THE DESCRIPTION

**Patent documents cited in the description**

- US 2017219215 A1
- US 2016353921 A1
- US 2015185712 A1
- US 2019320844 A1
- US 2017011649 A1

**Non-patent literature cited in the description**

- **ANONYMOUS.** *Free for*
- **ANONYMOUS.** *smart oven identifies your food and lets you watch it cook in real time via an internal camera - The Verge*
- **RIZVI, S. S. H. ; RAO, M. A.** Engineering properties of food. CRC Press, 1995, vol. 2